# EUROPEAN PATENT SPECIFICATION

(11) **EP 0 949 887 B1**
(45) Date of publication and mention of the grant of the patent: **29.11.2006**
(21) Application number: 97945561.5
(22) Date of filing: 07.10.1997
(51) Int. Cl.: A61B 17/70

(54) **A MODULAR POLYAXIAL LOCKING PEDICLE SCREW**
MODULAR AUFGEBAUTE, MEHRACHSIGE PEDIKELSCHRAUBE MIT VERRIEGELUNG
VIS PEDICULAIRE MODULAIRE POLYAXIALE DE VERROUILLAGE

(30) Priority: 09.10.1996 US 728017; 11.07.1997 US 799720
(43) Date of publication of application: 20.10.1999
(60) Divisional application: 05077314.2
(73) Proprietor: K2 Medical, L.L.C., Leesburg, Virginia 20175 (US)
(72) Inventor: ERRICO, Joseph, P., Far Hills, NJ 07931 (US); ERRICO, Thomas, J., Summit, NJ 07901 (US); RALPH, James, D., Oakland, NJ 07436 (US); TATAR, Steven, Montville, NJ 07045 (US)
(74) Representative: McCall, John Douglas
(86) International application number: PCT/US1997/018155
(87) International publication number: WO 1998/015233

(56) References cited:
- WO-A-96/29947
- DE-A- 19 512 709
- US-A- 4 946 458
- US-A- 5 344 422
- US-A- 5 486 174
- US-A- 5 501 684
- US-A- 5 575 792

## Description

### FIELD OF THE INVENTION

This invention relates generally to a polyaxial pedicle screw for use with orthopedic fixation systems having modular components. More particularly, the present invention relates to a screw for insertion into the vertebral bone having a shaft and a set of modular coupling elements which are polyaxially mounted thereto, via a stem member which is flexibly connected to the top portion of the shaft portion, therein enhancing the efficacy of the implant assembly by providing freedom of angulation among the rod, shaft and modular elements.

### DESCRIPTION OF THE PRIOR ART

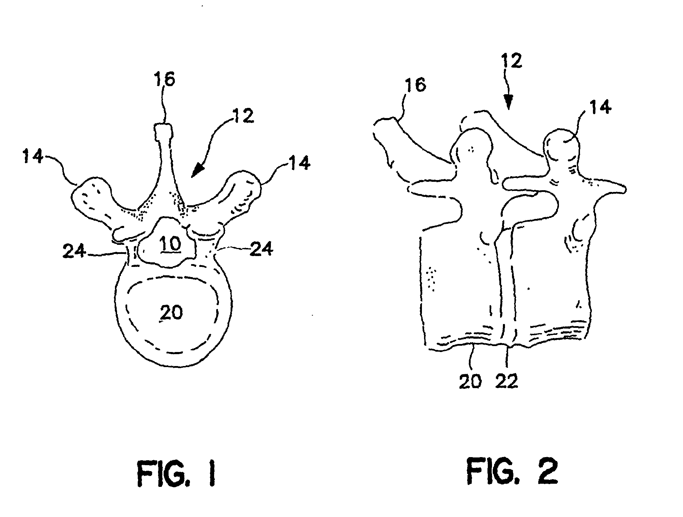

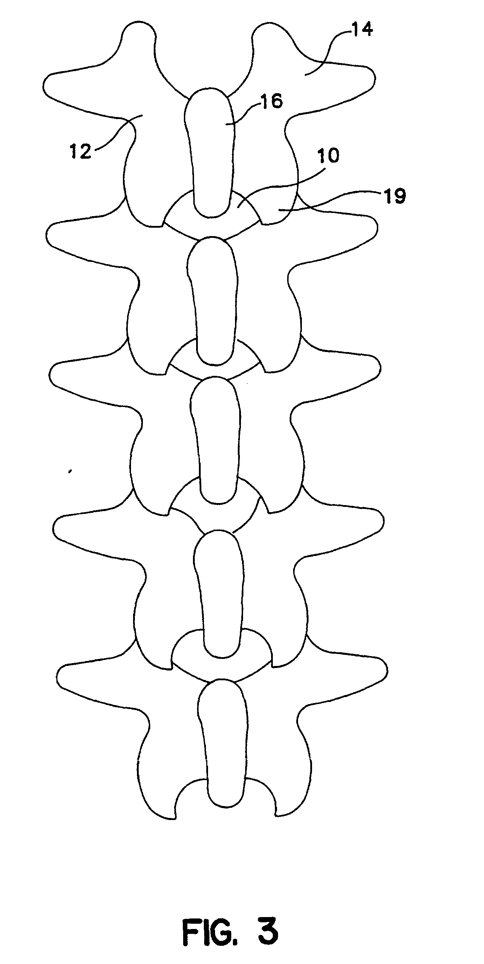

The bones and connective tissue of an adult human spinal column consists of more than 20 discrete bones coupled sequentially to one another by a tri-joint complex which consist of an anterior disc and the two posterior facet joints, the anterior discs of adjacent bones being cushioned by cartilage spacers referred to as intervertebral discs. Referring now to Figures 1, 2, and 3, top, side, and posterior views of a vertebral body, a pair of adjacent vertebral bodies, and a sequence of vertebral bodies are shown, respectively. The spinal cord is housed in the central canal 10, protected from the posterior side by a shell of bone called the lamina 12. The lamina 12 includes a rearwardly and downwardly extending portion called the spinous process 16, and laterally extending structures which are referred to as the transverse processes 14. The anterior portion of the spine comprises a set of generally cylindrically shaped bones which are stacked one on top of the other. These portions of the vertebrae are referred to as the vertebral bodies 20, and are each separated from the other by the intervertebral discs 22. The pedicles 24 comprise bone bridges which couple the anterior vertebral body 20 to the corresponding lamina 12.

The spinal column of bones is highly complex in that it includes over twenty bones coupled to one another, housing and protecting critical elements of the nervous system having innumerable peripheral nerves and circulatory bodies in close proximity. In spite of these complexities, the spine is a highly flexible structure, capable of a high degree of curvature and twist in nearly every direction. Genetic or developmental irregularities, trauma, chronic stress, tumors, and disease, however, can result in spinal pathologies which either limit this range of motion and/or threaten the critical elements of the nervous system housed within the spinal column. A variety of systems have been disclosed in the art which achieve this immobilization by implanting artificial assemblies in or on the spinal column. These assemblies may be classified as anterior, posterior, or lateral implants. As the classifications suggest, lateral and anterior assemblies are coupled to the anterior portion of the spine, which is the sequence of vertebral bodies. Posterior implants generally comprise pairs of rods, which are aligned along the axis which the bones are to be disposed, and which are then attached to the spinal column by either hooks which couple to the lamina or attach to the transverse processes, or by screws which are inserted through the pedicles.

"Rod assemblies" generally comprise a plurality of such screws which are implanted through the posterior lateral surfaces of the laminae, through the pedicles, and into their respective vertebral bodies. These screws are typically provided with upper portions which comprise coupling means, for receiving and securing an elongate rod therethrough. The rod extends along the axis of the spine, coupling to the plurality of screws via their coupling means. The rigidity of the rod may be utilized to align the spine in conformance with a more healthful shape.

It has been identified, however, that a considerable difficulty is associated with inserting screws along a misaligned curvature and simultaneously exactly positioning the coupling elements such that the rod receiving portions thereof are aligned so that the rod can be passed therethrough without distorting the screws. Attempts at achieving proper alignment with fixed screws is understood to require increased operating time, which is known to enhance many complications associated with surgery. Often surgical efforts with such fixed axes devices cannot be achieved, thereby rendering such instrumentation attempts entirely unsucessful.

US-A-4 946 458 describes a pedicle screw which comprises a shaft having a socket, a stem having a post portion and a ball portion which fits into said socket and a cuff to couple the shaft and the stem. The preamble to claim 1 of the present case is based on US-A-4,946,458.

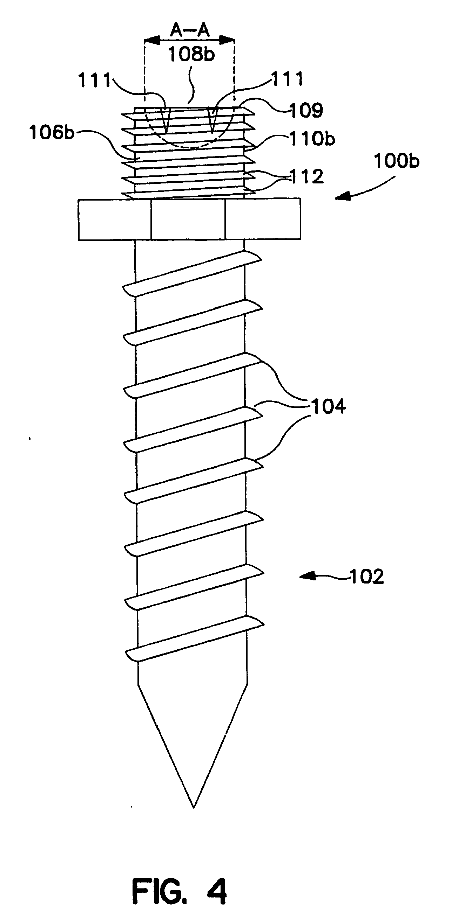

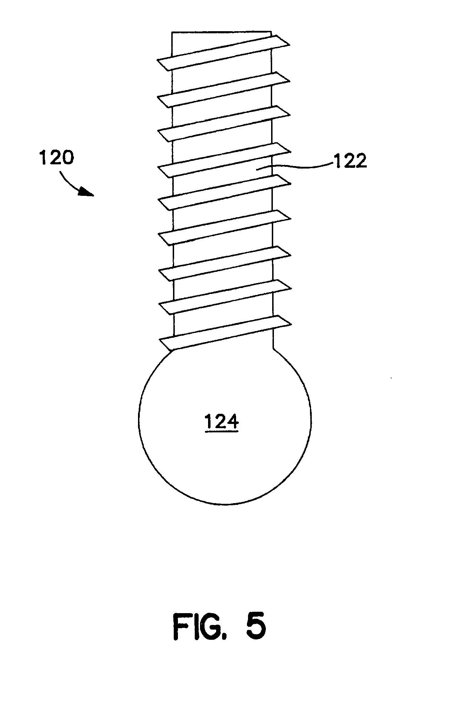

The art contains a variety of attempts at providing instrumentation which permit enhanced freedom for the surgeon with respect to aligning the screw and the rod, however, most are complex, inadequately reliable, and lack long-term durability. In addition, most generally lack the feature of being constructed to suit the specific anatomical requirements of every patient's spine. In particular, the Isola(TM) system, which is produced by Acromed, suffers from many of these failures in as much as it does not provide the surgeon to freely angulate the rod coupling means of the screw to meet the rod. More specifically, as illustrated in Figures 4 and 5, the Isola system consists of a shaft portion which is to be inserted into the patient's pedicle, the shaft having a threaded stem portion rigidly extending upwardly from the top of the shaft portion. (The interface of the shaft portion and the stem portion includes a hexagonally shaped annulus for engagement with a torque wrench to permit insertion.) Once the shaft and stem have been inserted, the surgeon threadably advances a number of spacer elements onto the stem portion (the spacer elements are threaded washers, some having a non-uniform thickness so as to provide an angular bias or tilt to the overall construct). Next the surgeon places the rod coupling means (which is slideably advanced axially onto the rod) onto the stem. The rod coupling means includes an elongated slot so that the specific position of the rod coupling means relative to the stem may be varied slightly. Once fully positioned, the surgeon secures the assembly together with a top locking nut.

While being modular so as to provide limited variability in the construct, the Isola system has a very limited ability to angulate (the stem is rigidly connected to the shaft portion), and what limited ability to angulate that it has entails the use of a plethora of non-uniformly thick spacer elements which are tedious to use in a surgical environment.

It is, therefore, the principle object of the present invention to provide a pedicle screw and coupling element assembly which provides a polyaxial freedom of implantation angulation with respect to rod reception.

### SUMMARY OF THE INVENTION

According to the present invention there is provided a modular pedicle screw assembly, comprising:
a shaft having a curvate socket formed in the top thereof, said socket being a recess having a constant radius of curvature and defining a surface which is greater in extent than a hemisphere;
a stem having an upper post portion and a lower ball portion, the ball portion having a larger diameter than the upper post portion, and said ball portion being shaped to nest and initially rotate in said curvate socket;
a cuff having a hollow cylindrical body having an opening in a top thereof having a diameter greater than that of the upper post portion and less than that of the ball portion, such that prior to positioning the cuff on the shaft the stem may polyaxially rotate relative to the shaft through a range of orientations including coaxial and non-coaxial ones, and such that after positioning the cuff on the shaft the ball portion is crush locked in the curvate socket, thereby preventing further motion of the stem relative to the shaft;
means for coupling said cuff on said shaft;
means for coupling a rod to the stem; and characterized by
at least one axial slot formed in the top of said shaft, said slot extending axially downward from said top of said shaft to a position which is lower than the maximum diameter of said curvate socket.

By use of the present invention one or more of the following may be achieved:-
(i) an assembly which comprises a reduced number of elements,
   and which correspondingly provides for expeditious implantation.
(ii) an assembly which is reliable, durable, and provides long term fixation support.

More particularly, the shaft portion includes an elongate shank portion having the threading of a bone screw (standard or otherwise, but suited for proper purchase of the bone). The uppermost portion of the shaft comprises a bowl-shaped recess which forms a socket. This socket portion is greater than a fully hemispherical recess. At least one axial slot needs to be provided so that the ball may be slipped into the socket. A small hexagonal bore may be disposed in the based of the bowl-shaped socket, coaxial with the shaft, so that a screw driving device (allen wrench, etc.) may be utilized to insert the shaft into the bone. Alternatively, a widened annular portion of the shaft, disposed between the two threadings may be hexagonal so as to permit the use of a torque wrench or other surgical tool. The axial surface of the shaft at the top (socket) end includes the second threading, for receiving and locking the cuff element thereto.

The stem comprises an elongate post portion having an enlarged ball (having a larger diameter than the post portion) formed at the bottom thereof; the enlarged ball having substantially the same radius of curvature as the bowl-shaped socket portion of the shaft. Therefore, when the ball is initially placed in the bowl-shaped socket, the stem may be positioned in a variety of different angulations relative to the axis of the shaft portion (through a polyaxial range of configurations from coaxial to substantially non-coaxial).

In a preferred embodiment the securing cuff comprises a hollow cylindrical body, having a threading on the bottom half (halves being defined relative to the through axis) of the interior surface thereof. This threading is designed to mate with the threading on the uppermost portion of the shaft element. The inner diameter of the open end of the top of the cuff is larger than the diameter of the post portion of the stem, but more narrow than the diameter of the ball formed at the bottom end of the stem. During assembly, the stem and shaft portions are initially held coaxial, with the ball of the stem in the socket of the shaft portion, while the securing cuff is advanced down along the post portion of the stem until the threadings of the cuff and the uppermost exterior surface of the shaft engage. Prior to final tightening, the stem and shaft are thereby held together by the cuff, but each may be angulated relative to the other by virtue of the ball and socket interface. (The total range of angulation is established by the relative diameters of the ball, the post, and the opening at the top of the cuff). Complete tightening of the cuff, however, causes the ball to be crushed into the socket of the shaft (and the tapered interior of the upper portion of the cuff), thereby preventing any further motion.

A portion of the exterior of the cuff preferably comprises a hexagonally angled surface contour, such that the cuff may be easily engaged by a torque wrench. (It shall be understood that any one of a variety of such surface contours or other means may be employed equivalently). The upper exterior of the cuff, however, is rounded (with a constant radius of curvature) so that it provides a curvate profile. This permits the secure engagement of similarly rounded spacer elements relative to the top of the cuff element independent of the angular orientation of the post portion of the stem relative to the cuff and shaft (the cuff and shaft remain coaxial).

More particularly, inasmuch as it is still desirable to permit variable positioning of the rod coupling means along the axis of the stem, in addition to the angular variability provided by the polyaxial shaft-stem-cuff assembly, spacer elements may also be utilized. The spacers are annular elements having a diameter which is equivalent to that of the post portion (and are preferably threaded). The bottom surfaces of the spacer elements are concave, having a radius of curvature equal to that of the upper surface of the cuff. As stated above, this mutual contour permits the spacer to seat securely against the cuff independent of the angulation of the stem. The upper surface of the spacer element is convex, having an equivalent radius of curvature, such that multiple spacers may be nested.

It shall be understood that the advancing the spacer elements downwardly on the stem, and into contact with the cuff, and subsequent tightening, causes an increase in the total locking force applied to the ball in the socket (the ball is pulled into tighter contact with the socket-shaped interior of the cuff). In order to threadably advance the spacer elements easily, the outer lateral surface of the elements are contoured so as to be engageable by a torque wrench; e.g. having a hexagonal shape.

The rod coupling element of this assembly comprises a flat portion having an elongate hole therethrough for coupling to the stem, and a tubular portion which may be slidably advanced along the rod into the proper position. Once in the proper position, the rod coupling means is locked to the rod by a set screw in order to prevent further movement relative to the rod. The elongate hole in the flat portion is elongate in nature so that distance from the rod to the stem may be varied. This element further includes the concave conformation on the underside thereof so that it may nest securely on the upper surface of either a spacer or directly on the cuff (in the case wherein no spacer is used).

The first step in the process of implanting this invention is to pre-drill the appropriate site in the pedicle to receive the shaft. The shaft is then driven into the vertebral body. The cuff is then advance down the stem portion until it reaches the ball or socket portion. The ball and socket portions are then placed together such that they may rotate relative to one another, and the threadings on the top of the shaft and on the interior of the cuff are engaged. The stem is then angulated into the appropriate position, and the cuff is locked down, thereby securing the stem relative to the shaft. The rod coupling element is then slidably advanced along the rod into the appropriate position, and the stem placed in the elongate hole thereof. The set screw of the rod coupling element is engaged to lock thereto. (Spacers are threadably advanced onto the stem prior to the insertion of the stem through the elongate hole, if it is determined that they are necessary). Once the assembly has been properly set, the top locking nut is advanced downwardly along the stem and into position against the top of the rod coupling element, thereby preventing any lateral or axial movement of the stem within the elongate hole.

Multiple screw and coupling element assemblies are generally necessary to complete the full array of anchoring sites for the rod immobilization system, however, the screw and coupling element assembly of the present invention is designed to be compatible with alternative rod systems so that, where necessary, the present invention may be employed to rectify the failures of other systems the implantation of which may have already begun.

### BRIEF DESCRIPTION OF THE FIGURES

Figure 1 is a top view of a human vertebra, which is representative of the type for which the present invention is useful for coupling thereto a rod apparatus;
Figure 2 is a side view of a pair of adjacent vertebrae of the type shown in Figure 1;
Figure 3 is a posterior view of a sequence of vertebrae of the type shown in Figures 1 and 2;
Figure 4 is a side cross section view of a threaded shaft which is an aspect of the present invention;
Figure 5 is a side view of a stem portion, having a ball formed at the bottom thereof, which is an aspect of the present invention;
Figure 6 is a side cross section view of cuff of the present invention;
Figure 7 is a side cross section view of a spacer element which is an aspect of the present invention;
Figure 8 is a perspective view of a rod coupling element which is an aspect of the present invention;
Figure 9 is a side cross-sectional view of the top locking nut of the present invention;
Figure 10 is a side view of a fully assembled modular polyaxial pedicle screw of the present invention;

### DETAILED DESCRIPTION OF THE PREFERRED EMBODIMENT

While the present invention will be described more fully hereinafter with reference to the accompanying drawings, in which particular embodiments and methods of implantation are shown, it is to be understood at the outset that persons skilled in the art may modify the invention herein described while achieving the functions and results of this invention. Accordingly, the descriptions which follow are to be understood as illustrative and exemplary of specific structures, aspects and features within the broad scope of the present invention and not as limiting of such broad scope.

More particularly, referring now to Figure 4, the modular polyaxial pedicle screw of the present invention first comprises a threaded shaft portion 100b which is inserted into the pedicle. Shaft 100b includes a lower shank portion 102 which include a bone screw threading 104. (This threading 104 may be standard or otherwise, but is in any case suited for necessary purchase of bone)

The top 106b of the shaft 100b may comprise a socket 108b which is greater than a hemispherical section. In such a socket 108b, the upper lip 109 therefore should include at least one axial slot 111 which extends below the maximum diameter A-A, and which may expand and contract in accordance with the application of a radial force on the upper lip 109.

The axial surface of the upper end 110b of the shaft 100b (around the socket 108b) includes a second threading 112. A widened hexagonal annulus 114, which is integrally formed with the shaft 100b, and is disposed between the threaded shank 102 and upper portion 110b, is provided so that a suitable torque wrench may be employed to drive the shaft 100b into the vertebral body through the pedicle.

Referring now to Figure 5, a stem portion 120 is shown in a side view. More specifically, the stem 120 comprises an elongate threaded post portion 122 and an enlarged ball 124. The ball 124 has a larger diameter than the post 122. The ball 124 has substantially the same radius of curvature as the bowl-shaped socket 108 portion of the shaft 100. This mutual dimension permits the ball 124 to rotate freely in the socket 108 once the ball 124 is placed therein, thus permitting the stem 120 to be angulated relative to the shaft 100 (through a polyaxial range from coaxial to substantially non-coaxial).

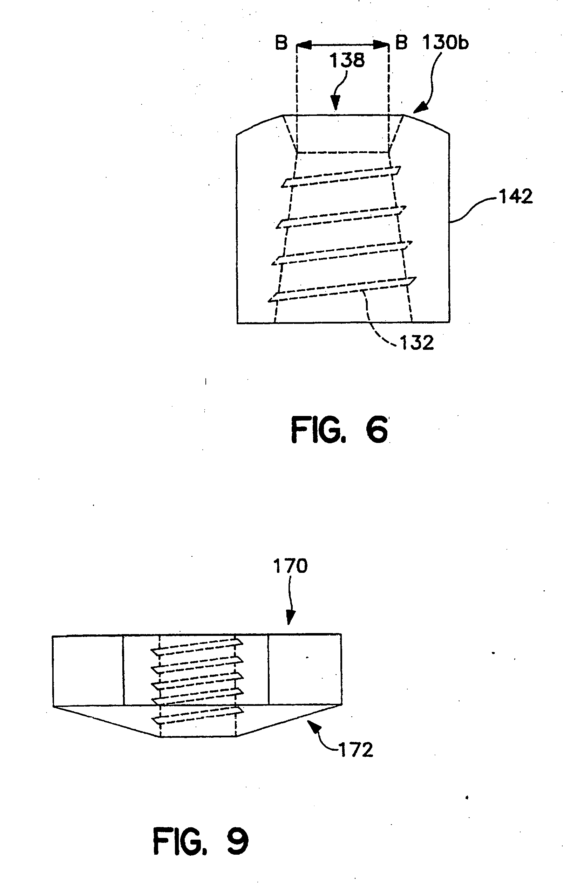

Referring to Figure 6, the mutual engagement of the shaft 100b with the stem 120 is provided by corresponding cuff 130b, shown in side cross section view. The cuff 130b is a hollow cylinder and has a threading 132 on the bottom half of the interior surface 134 thereof. This threading 132 is designed to mate with the threading 112 on the uppermost portion 110b of the shaft element 100b. The lower threaded portion of cuff 130b may include a reverse taper, i.e. narrower at the top, in order to provide an inwardly directed radial force onto the upper lip 109. The inner diameter B-B of the open end 138 of the top of the cuff 130b is larger than the diameter of the threaded post portion 122 of the stem 120, but more narrow than the diameter of the ball 124.

During assembly, the stem 120 and shaft 100b portion are initially held coaxial, with the ball 124 of the stem 120 in the socket 108b of the shaft 100b (the ball 124 being snapped into the socket 108b by applying a downward force whereby the at least one slot 111 expands), while the securing cuff 103b is advanced down along the post portion of the stem until the threadings 132, 112 of the cuff 130b and the uppermost exterior surface 110b of the shaft, respectively, engage. Prior to final tightening, the stem 120 and shaft 100b are thereby loosely held together by the cuff 130b, respectively, but each may be angulated relative to the other by virtue of the ball 124 and socket 108b interface. The total range of angulation is established by the relative diameters of the ball 124, the post 122, (the upper lip 109 of the socket 108b), and the opening 138 at the top of the cuff. Complete tightening of the cuff 130b, however, causes the ball 124 to be crushed into the socket 108b, thereby preventing any further motion.

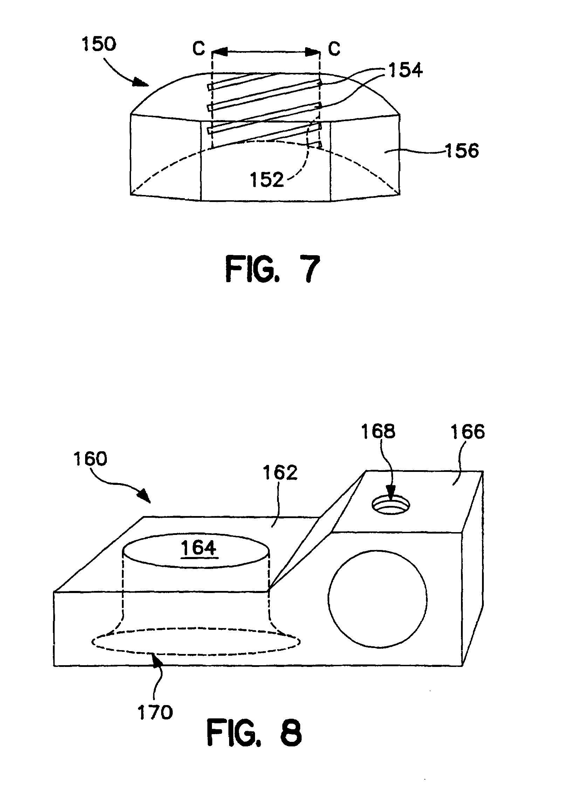

A portion of the exterior 140 of the cuff 130b comprises a hexagonally angled surface contour, such that the cuff 130b may be easily engaged by a torque wrench. In addition, the upper exterior 142 of the cuff 130b, however, is rounded (with a constant radius of curvature) so that it provides a curvate profile. This permits the secure engagement of similarly rounded spacer elements (see Figure 7, and related description hereinbelow) relative to the top of the cuff 130b independent of the angular orientation of the post 122 of the stem 120 relative to the cuff 130b and shaft 100b.

More particularly, with reference to Figure 7, inasmuch as it may be desirable to vary the axial position of the rod coupling means (see Figure 8, and related description hereinbelow) along the post 122, spacer elements 150 are provided. The spacers 150 are annular elements having an inner diameter C-C which is equivalent to that of the post 122. In the illustrated embodiment, the inner surface 152 includes a threading 154 which is engageable with the threading of the post 122. (It is not necessary for the spacers 150 to be threaded, however, such a threading may provide additional downward force on the cuff 130b to further secure the locking of the stem 120 and the shaft 100b). The bottom surface of the spacer 150 is concave, having a radius of curvature equal to that of the tapered upper portion 142 of the cuff 130b. This mutual contour permits the spacer 150 to seat securely against the cuff 130b independent of the angulation of the stem 120. The upper surface 142 of the spacer 150 is convex, having an equivalent radius of curvature, such that multiple spacers 150 may be nested. In order that the threaded spacer 150 may be advanced easily along the threaded post 122, the outer lateral surface 156 is contoured so as to be engageable by a torque wrench; e.g., having a hexagonal shape.

Referring now to Figure 8, the rod coupling element 160 of this assembly comprises a flat portion 162 having an elongate hole 164 therethrough for coupling to the stem 120. The elongate hole 164 has a width equal to that of the post 122, but is elongated to permit variable lateral placement of the post 122 relative to the rod 200. The edge (not seen in this illustration) of the elongated hole 164, on the underside of the flat portion, is concavely tapered so as to ideally receive the curvate upper portion of the spacer 150 or the cuff 130b.

The rod coupling element 160 further includes a tubular portion 166, the axis of the tube is substantially perpendicular to the elongated axis of the hole 164. The rod coupling element 160 is positioned on the rod 200 by slideably advancing it therealong. Once in the proper position, the rod coupling element 160 is locked to the rod 200 by a set screw 168 in order to prevent further movement relative to the rod. In addition, this element 160 further includes a concave underside conformation 170 such that it may nest securely on the upper surface of either a spacer 150 or directly on the cuff 130a,130b (in the case wherein no spacer 150 is used).

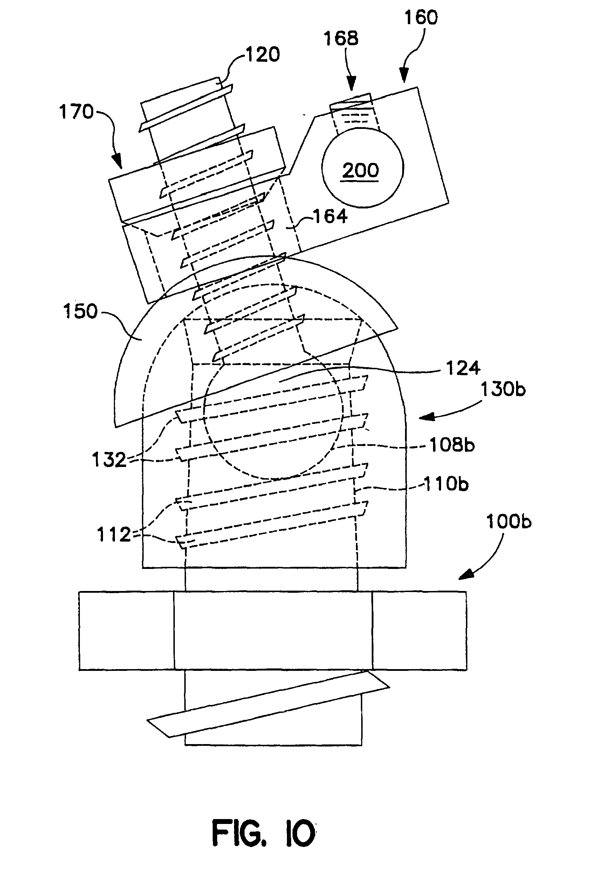

Referring to Figure 9, a top locking nut 170 is utilized to lock the rod coupling element 160 onto the post 122. More particularly, the top locking nut 170 has a bottom surface 172 which is ideally suited to engage and hold the post 122 and the rod coupling element 160 from axial or lateral movement. Specifically, the top locking nut 170 is designed to apply a downward pressure which is sufficient to lock the cuff 130b into the tapered curvate edge of the elongate hole 164 such that the friction locking force of the spacer 150 or cuff 130b thereagainst is sufficient to hold the rod coupling element 160 from lateral, or axial, movement.

Referring now to Figure 10, the steps of implanting this assembly is described. First, a hole is pre-drilled in the appropriate site in the pedicle in order to receive the shaft 100b. The shaft 100b is then driven into the vertebral body. The cuff 130b is then advanced down the stem 120 until it reaches the ball 124. The ball 124 is then placed (or snapped) into the socket 108b, and the threadings 112, 132 on the upper portion 110b of the shaft 100b and on the interior of the cuff 130b, respectively, are engaged. The post 122 is then angulated into the appropriate position, and the cuff 130b is locked down, thereby securing the stem 120 relative to the shaft 130b. (The ball 124 is crushed into the socket 108b by virtue of the socket itself being collapsed onto the ball 124.) If necessary, spacers 150 are positioned above the cuff 130b on the post 122. The rod coupling element 160 is then slideably advanced along the rod 200 into the appropriate position, and the post 122 is placed in the elongate hole 164 thereof. The set screw 168 of the rod coupling element 160 is engaged to lock it to the rod 200. Finally the top locking nut 180 is advanced downwardly along the post 122 and into position against the rod coupling element 160, thereby preventing any lateral or axial movement of the post 122 within the elongate hole 164.

A complete posterior rod implant system includes at least two, and generally four or more, screw assemblies. However, this assembly, as set forth above, may also be used in conjunction with other screw assemblies in the art wherein there is an immediate need for the beneficial properties of this assembly to correct deficiencies in the other assemblies. Therefore, it is anticipated that this modular polyaxial pedicle screw may be used individually, or in conjunction with others

A complete posterior rod implant system includes at least two, and generally four or more, screw assemblies. However, this assembly, as set forth above, may also be used in conjunction with other screw assemblies in the art wherein there is an immediate need for the beneficial properties of this assembly to correct deficiencies in the other assemblies. Therefore, it is anticipated that this modular polyaxial pedicle screw may be used individually, or in conjunction with others.

While there has been described and illustrated embodiments of a modular polyaxial pedicle screw assembly for use with posterior spinal rod implantation apparatus, it will be apparent to those skilled in the art that variations and modifications are possible without deviating from the broad principle of the present invention. The present invention shall, therefore, be limited solely by the scope of the claims appended hereto.

## Claims

1. A modular polyaxial pedicle screw assembly, comprising:
a shaft (100b) having a curvate socket (108b) formed in the top (106b) thereof, said socket being a recess having a constant radius of curvature and defining a surface which is greater in extent than a hemisphere;
a stem (120) having an upper post portion (122) and a lower ball portion (124), the ball portion (124) having a larger diameter than the upper post portion (122), and said ball portion (124) being shaped to nest and initially rotate in said curvate socket (108b);
a cuff (130b) having a hollow cylindrical body having an opening (138) in a top thereof having a diameter greater than that of the upper post portion (122) and less than that of the ball portion (124), such that prior to positioning the cuff (130b) on the shaft (100b) the stem (120) may polyaxially rotate relative to the shaft (100b) through a range of orientations including coaxial and non-coaxial ones, and such that after positioning the cuff (130b) on the shaft (100b) the ball portion (124) is crush locked in the curvate socket (108b), thereby preventing further motion of the stem (120) relative to the shaft (100b);
means (112, 132) for coupling said cuff (130) on said shaft;
means (160) for coupling a rod to the stem; and **characterized by**
at least one axial slot (111) formed in the top of said shaft (100b), said slot (111) extending axially downward from said top (106b) of said shaft (100b) to a position which is lower than the maximum diameter of said curvate socket (108b).

2. An assembly as claimed in claim 1, in which a threading (112) is disposed on the upper circumferential surface of said shaft (100b);
and said cuff (130b) includes a threading (132) on a lower interior surface thereof which threading (132) is mateable with said threading (112) on said upper circumferential surface of said shaft (100b).

3. An assembly as claimed in claim 2, wherein the shaft (100b) includes a second threading (104) disposed on a lower circumferential surface thereof.

4. An assembly as claimed in any one of the preceding claims, wherein the shaft (100b) includes a lower interior surface which is tapered upwardly and radially inwardly.

5. An assembly as claimed in claim 2, wherein said shaft (100b) further comprises an annulus portion integrally formed therewith which is positioned beneath the threaded upper circumferential portion, said annulus having a hexagonal outer conformation.

6. An assembly as claimed in claim 2, wherein said upper post portion (122) comprises a surface threading.

7. An assembly as claimed in claim 2, wherein said cuff (130b) includes an external circumferential surface portion which defines a hexagonal conformation.

8. An assembly as claimed in claim 2, wherein the cuff (130b) includes an upper exterior surface and a top exterior surface which are mutually axially tapered to exhibit a rounded conformation.

9. An assembly as claimed in claim 8, further comprising at least one spacer element (150) disposed between the cuff (130b) and the means (160) for coupling a rod to said stem (120).

10. An assembly as claimed in claim 9, wherein said at least one spacer element (150) comprises a washer having a concave lower surface and a convex upper surface, said concavity and convexity of said upper and lower surfaces having the equivalent absolute curvature as said exterior and top surfaces of said cuff (130b).

11. An assembly as claimed in claim 10, wherein said upper post portion (122) of said stem (120) includes an exterior surface threading and wherein said interior circumferential surface of said at least one spacer element also includes a threading.

12. An assembly as claimed in claim 11, wherein said at least one spacer element (150) comprises an exterior circumferential conformation which defines a hexagonal shape.

13. An assembly as claimed in claim 2, wherein said means (160) for coupling a rod to said stem comprises a rod coupling element which is mountable on said upper post portion (122) of said stem (120) and top locking nut (170) securable to said upper post portion (122), above said rod coupling element, thereby securing said rod coupling element between the top locking nut (170) and said cuff (130b).

14. An orthopaedic implant device for use in a spine, comprising:
at least one rod positioned in parallel with the elongate axis of the spine;
a plurality of pedicle screw assemblies for securely coupling said at least one rod to said spine, at least one of said pedicle screws being a modular polyaxial pedicle screw as claimed in any one of the preceding claims.

## Patentansprüche

1. Modulare polyaxiale Pedikelschraubenbaugruppe, die umfasst:
einen Schaft (100b) mit einer Krümmungsfassung (108b) ausgebildet im oberen Teil (106b) desselben, wobei die Fassung eine Ausnehmung mit einem konstanten Krümmungsradius ist und eine Oberfläche begrenzt, die einen größeren Umfang als eine Halbkugel aufweist;
einen Stiel (120) mit einem oberen Pfostenteil (122) und einem unteren Kugelteil (124), wobei der Kugelteil (124) einen größeren Durchmesser als der obere Pfostenteil (122) aufweist, und der Kugelteil (124) geformt ist, um in die Krümmungsfassung (108b) eingesteckt zu werden und zu Beginn in dieser zu rotieren;
eine Manschette (130b) mit einem hohlen zylindrischen Körper, in dessen Oberseite eine Öffnung (138) mit einem größeren Durchmesser als dem des oberen Pfostenteils (122) und kleiner als dem des Kugelteils (124) vorgesehen ist, so dass vor Positionierung der Manschette (130b) auf dem Schaft (100b) der Stiel (120) polyaxial in Bezug zum Schaft (100b) über einen Bereich von Ausrichtungen rotieren kann, die Koaxiale und Nichtkoaxiale einschließen, und so dass, nach Positionierung der Manschette (130b) auf dem Schaft (100b), der Kugelteil (124) in der Krümmungsfassung (108b) quetschverriegelt wird, wodurch weitere Bewegung des Stiels (120) in Bezug zum Schaft (100b) verhindert wird;
ein Mittel (112, 132) zum Koppeln der Manschette (130) auf den Schaft;
ein Mittel (160) zum Koppeln eines Stab an den Stiel; und die **gekennzeichnet ist durch**
mindestens einen axialen Schlitz (111) ausgebildet in dem oberen Teil des Schafts (100b), wobei der Schlitz (111) sich axial nach unten von dem oberen Teil (106b) des Schafts (100b) zu einer Position erstreckt, die niedriger als der maximale Durchmesser der Krümmungsfassung (108b) ist.

2. Baugruppe nach Anspruch 1, bei der ein Gewinde (112) auf der oberen Umfangsfläche des Schafts (100b) angeordnet ist;
und die Manschette (130b) ein Gewinde (132) an einer unteren Innenfläche derselben aufweist, welches Gewinde (132) mit dem Gewinde (112) auf der obere Umfangsfläche des Schafts (100b) in Eingriff gebracht werden kann.

3. Baugruppe nach Anspruch 2, bei der der Schaft (100b) ein zweites Gewinde (104) angeordnet auf einer unteren Umfangsfläche desselben umfasst.

4. Baugruppe nach einem der vorhergehenden Ansprüche, bei der der Schaft (100b) eine untere Innenfläche umfasst, die sich nach oben und radial nach innen verjüngt.

5. Baugruppe nach Anspruch 2, bei der der Schaft (100b) weiter einen Ringteil einstückig mit demselben ausgebildet aufweist, welcher unter dem oberen Gewindeumfangsteil positioniert ist, wobei der Ring eine hexagonale Außengestaltung aufweist.

6. Baugruppe nach Anspruch 2, bei der der obere Pfostenteil (122) ein Oberflächengewinde aufweist.

7. Baugruppe nach Anspruch 2, bei der die Manschette (130b) einen äußeren Umfangsflächenteil umfasst, der eine hexagonale Gestaltung definiert.

8. Baugruppe nach Anspruch 2, bei der die Manschette (130b) eine obere Außenfläche und eine oberste Außenfläche umfasst, die sich zueinander axial verjüngen, um eine gerundete Gestaltung aufzuweisen.

9. Baugruppe nach Anspruch 8, die weiter mindestens ein Abstandselement (150) aufweist, das zwischen der Manschette (130b) und dem Mittel (160) zum Koppeln eines Stabs an den Stiel (120) angeordnet ist.

10. Baugruppe nach Anspruch 9, bei der das mindestens eine Abstandselement (150) eine Zwischenscheibe mit einer konkaven unteren Fläche und einer konvexen oberen Fläche aufweist, wobei Konkavität und Konvexität der oberen und unteren Oberfläche die äquivalente absolute Krümmung wie die äußere und oberste Oberfläche der Manschette (130b) haben.

11. Baugruppe nach Anspruch 10, bei der der obere Pfostenteil (122) des Stiels (120) ein Außenflächengewinde umfasst, und bei der die inneren Umfangsfläche des mindestens einen Abstandselements auch ein Gewinde umfasst.

12. Baugruppe nach Anspruch 11, bei der das mindestens ein Abstandselement (150) eine äußere Umfangsgestaltung aufweist, die eine hexagonale Form definiert.

13. Baugruppe nach Anspruch 2, bei der das Mittel (160) zum Koppeln eines Stabs an den Stiel ein Stabkopplungselement, das an dem oberen Pfostenteil (122) des Stiels (120) angebracht werden kann, und eine obere Sicherungsmutter (170) aufweist, die an dem oberen Pfostenteil (122) über dem Stabkopplungselement befestigt werden kann, wodurch das Stabkopplungselement zwischen der oberen Sicherungsmutter (170) und der Manschette (130b) befestigt wird.

14. Orthopädische Implantatvorrichtung zum Gebrauch in einem Rückgrat, die umfasst:
mindestens einen Stab, der parallel zu der Längsachse des Rückgrats positioniert ist;
eine Mehrzahl von Pedikelschraubenbaugruppen zum sicheren Koppeln des mindestens einen Stabs an dem Rückgrat, wobei mindestens eine der Pedikelschrauben eine modulare polyaxiale Pedikelschraube nach einem der vorhergehenden Ansprüche darstellt.

## Revendications

1. Ensemble de vis pédiculaire modulaire polyaxiale, comportant :
un arbre (100b) ayant une douille courbe (108b) réalisée dans la partie supérieure (106b) de celui-ci, ladite douille étant un évidement ayant un rayon de courbure constant et définissant une surface qui est supérieure en étendue par rapport à un hémisphére ;
une tige (120) ayant une portion supérieure de type montant (122) et une portion inférieure de type boule (124), la portion de type boule (124) ayant un diamètre supérieur par rapport à la portion supérieure de type montant (122), et ladite portion de type boule (124) ayant une forme lui permettant de s'emboîter et au départ de pivoter dans ladite douille courbe (108b) ;
une coiffe (130b) ayant un corps cylindrique creux ayant une ouverture (138) dans une partie supérieure de celle-ci ayant un diamètre supérieur par rapport à celui de la portion supérieure de type montant (122) et inférieur par rapport à celui de la portion de type boule (124), de telle manière que, avant le positionnement de la coiffe (130b) sur l'arbre (100b), la tige (120) peut pivoter de manière polyaxiale par rapport à l'arbre (100b) selon une gamme d'orientations y compris des orientations coaxiales et non coaxiales, et de telle manière que, après le positionnement de la coiffe (130b) sur l'arbre (100b), la portion de type boule (124) est verrouillée par écrasement dans la douille courbe (108b), empêchant de ce fait tout autre mouvement de la tige (120) par rapport à l'arbre (100b) ;
des moyens (112, 132) permettant d'accoupler ladite coiffe (130) sur ledit arbre ;
un moyen (160) permettant d'accoupler une barre sur la tige ; et **caractérisé par**
au moins une fente axiale (111) réalisée dans la partie supérieure dudit arbre (100b), ladite fente (111) se prolongeant de manière axiale vers le bas en provenance de ladite partie supérieure (106b) dudit arbre (100b) jusqu'à une position qui est inférieure par rapport au diamètre maximum de ladite douille courbe (108b).

2. Ensemble selon la revendication 1, dans lequel un filetage (112) est disposé sur la surface circonférentielle supérieure dudit arbre (100b) ;
et ladite coiffe (130b) comprend un filetage (132) sur une surface intérieure inférieure de celle-ci, filetage (132) qui est en mesure de recevoir ledit filetage (112) sur ladite surface circonférentielle supérieure dudit arbre (100b).

3. Ensemble selon la revendication 2, dans lequel l'arbre (100b) comprend un deuxième filetage (104) disposé sur une surface circonférentielle inférieure de celui-ci.

4. Ensemble selon l'une quelconque des revendications précédentes, dans lequel l'arbre (100b) comprend une surface intérieure inférieure qui est amincie vers le haut et de manière radiale vers l'intérieur.

5. Ensemble selon la revendication 2, dans lequel ledit arbre (100b) comprend par ailleurs une portion de type anneau réalisée de manière intégrée avec celui-ci qui est positionnée en dessous de la portion circonférentielle supérieure filetée, ledit anneau ayant une conformation extérieure hexagonale.

6. Ensemble selon la revendication 2, dans lequel ladite portion supérieure de type montant (122) comporte un filetage au niveau de sa surface.

7. Ensemble selon la revendication 2, dans lequel ladite coiffe (130b) comprend une portion de surface circonférentielle externe qui définit une conformation hexagonale.

8. Ensemble selon la revendication 2, dans lequel la coiffe (130b) comprend une surface extérieure supérieure et une surface extérieure de partie supérieure qui sont mutuellement amincies de manière axiale afin de présenter une conformation arrondie.

9. Ensemble selon la revendication 8, comportant par ailleurs au moins un élément de type entretoise (150) disposé entre la coiffe (130b) et le moyen (160) permettant d'accoupler une barre sur ladite tige (120).

10. Ensemble selon la revendication 9, dans lequel ledit au moins un élément de type entretoise (150) comporte une rondelle ayant une surface inférieure concave et une surface supérieure convexe, ladite concavité et ladite convexité de ladite surface supérieure et de ladite surface inférieure ayant la courbure absolue équivalente telle que ladite surface extérieure et ladite surface de partie supérieure de ladite coiffe (130b).

11. Ensemble selon la revendication 10, dans lequel ladite portion supérieure de type montant (122) de ladite tige (120) comprend un filetage au niveau de sa surface extérieure et dans lequel ladite surface circonférentielle intérieure dudit au moins un élément de type entretoise comprend également un filetage.

12. Ensemble selon la revendication 11, dans lequel ledit au moins un élément de type entretoise (150) comporte une conformation circonférentielle extérieure qui définit une forme hexagonale.

13. Ensemble selon la revendication 2, dans lequel ledit moyen (160) permettant d'accoupler une barre sur ladite tige comporte un élément d'accouplement de barre qui est en mesure d'être monté sur ladite portion supérieure de type montant (122) de ladite tige (120) et un écrou de verrouillage de partie supérieure (170) en mesure d'être assujetti sur ladite portion supérieure de type montant (122), au-dessus dudit élément d'accouplement de barre, assujettissant de ce fait ledit élément d'accouplement de barre entre l'écrou de verrouillage de partie supérieure (170) et ladite coiffe (130b).

14. Dispositif de type prothèse orthopédique destiné à être utilisé dans une colonne vertébrale, comportant :
au moins une barre positionnée en parallèle par rapport à l'axe allongé de la colonne vertébrale ;
une pluralité d'ensembles de vis pédiculaires permettant d'accoupler solidement ladite au moins une barre à ladite colonne vertébrale, au moins une desdites vis pédiculaires étant une vis pédiculaire modulaire polyaxiale selon l'une quelconque des revendications précédentes.
